# EUROPÄISCHE PATENTSCHRIFT

(11) **EP 1 515 755 B1**
(45) Veröffentlichungstag und Bekanntmachung des Hinweises auf die Patenterteilung: **02.01.2008**
(21) Anmeldenummer: 03732573.5
(22) Anmeldetag: 13.06.2003
(51) Int. Cl.: A61L 2/18, A01N 33/02, A01N 33/08, A01N 33/12, A01N 37/18

(54) **Verfahren zur Verringerung des Wirkstoffverlustes**
Process for the reduction of loss of active substances
Procédé pour réduire la perte de substances actives

(30) Priorität: 22.06.2002 DE 10227872
(43) Veröffentlichungstag der Anmeldung: 23.03.2005
(73) Patentinhaber: ECOLAB INC., St. Paul, MN 55102-1390 (US)
(72) Erfinder: BIERING, Holger, 41516 Grevenbroich (DE); FAUBEL, Heiko, 42929 Wermelskirchen (DE); GLASMACHER, Rudolf, 40789 Monheim (DE); HEIDE, Veronika, 41542 Dormagen (DE)
(74) Vertreter: Polypatent
(86) Internationale Anmeldenummer: PCT/EP2003/006274
(87) Internationale Veröffentlichungsnummer: WO 2004/000373

(56) Entgegenhaltungen:
- EP-A- 1 126 014
- WO-A-94/27436
- WO-A-98/56886
- DE-A- 10 054 020
- FR-A- 2 622 397
- US-A- 4 661 523
- US-A- 5 929 016
- US-B1- 6 258 368

## Beschreibung

Die im Folgenden beschriebene Erfindung liegt auf dem Gebiet der Reinigung und Desinfektion von harten Oberflächen. Die Reinigung und Desinfektion der Oberflächen wird mittels Reinigungstextil und einer wässrigen Zubereitung, die Desinfektionsmittel und Additiv enthält, durchgeführt. Die Erfindung betrifft ein Verfahren zur Verringerung des Wirkstoffverlustes von Desinfektionsmittellösungen unter Anwendung eines Konzentrates.

Die Reinigung und Pflege von Oberflächen ist in hygienisch besonders anspruchsvollen Bereichen notwendig. Hierbei werden in regelmäßigen Abständen anhaftende Mikroorganismen möglichst weitgehend eliminiert. Es sind beispielsweise im Bereich der Lebensmittelindustrie und der Großküchen, aber insbesondere im medizinischen Bereich, vor allem in Arztpraxen und Krankenhäusern, besonders hohe Standards anzuwenden. Um die Reinigung und Desinfektion besonders effizient zu gestalten, hat es nicht an Versuchen gefehlt, eine Optimierung der Reinigung und Desinfektion durchzuführen.

US-6,258,368 offenbart antimikrobielle Wischlösungen für Tücher zur Hautreinigung. Die antimikrobielle Zusammensetzung besteht u. a. aus 0,0001 - 5 Gew.-% einer antimikrobiell aktiven Verbindung. Spezifische Verbindungen werden offenbart, insbesondere Phenole, Phenol-Derivate und Aldehyde. Weiterhin können quartäre Amoniumverbindungen in der Zusammensetzung enthalten sein.

In der DE 199 18 475 A1 ist ein Verfahren zur desinfizierenden Pflege von Fußböden offenbart. Die Aufgabe dieser Erfindung bestand darin, in bis dahin unerreichter Weise die breitwirksamen antimikrobiellen Wirkstoffe auf Aminbasis ohne Einbuße der Wirksamkeit in einem Verfahren gleichzeitig mit der reinigenden Pflege von Fußböden zu kombinieren. Bei der Durchführung dieses Verfahrens werden die Fußböden mit der verdünnten wässrigen Zubereitung gewischt. Das Wischen kann mit Hilfe von weichen, vorzugsweise saugfähigen Gegenständen, beispielsweise Bürsten, Tüchern, Flachwischbezügen und Schwämmen erfolgen und kann manuell oder mit Hilfe geeigneter Maschinen durchgeführt werden. Hierbei kann das Aufbringen der verdünnten wässrigen Zubereitung, bestehend aus Desinfektionsmittel und Reinigungsmittel, auch getrennt vom anschließenden Wischvorgang, beispielsweise durch Aufsprühen, erfolgen. Wenn eine verstärkte Reinigung gewünscht wird, können auch zunächst größere Mengen an wässriger Zubereitung ausgebracht und die überschüssigen Mengen nach dem Wischen zusammen mit dem abgelösten Schmutz wieder vom Fußboden aufgenommen werden. Die auf dem Fußboden verbleibende Menge an wässriger Zubereitung lässt man eintrocknen, wobei sich der gewünschte Pflegefilm auf der gereinigten und desinfizierten Oberfläche bildet.

An die Durchführung der Desinfektion von Fußböden und harten Oberflächen, insbesondere in Krankenhäusern und im sonstigen medizinischen Bereich werden aufgrund der erhöhten Hygienebestimmungen jedoch höhere Anforderungen gestellt. In diesen Bereichen haben sich nunmehr Verfahren durchgesetzt, die gewährleisten, dass es nicht zu einer Keimverschleppung durch Benutzung der selben Reinigungstextilien in verschiedenen Räumen kommen kann. Bewährte Verfahren sind insbesondere das Zwei-Bezugs-Verfahren und das Ein-Bezugs-Verfahren. Im Zwei-Bezugs-Verfahren wird eine definierte Menge Desinfektionsmittel pro Flächeneinheit manuell aufgegeben, mit dem ersten Flachwischbezug verteilt und danach mit einem zweiten Bezug die überschüssige Flüssigkeitsmenge aufgenommen.

Im Ein-Bezugs-Verfahren wird das Reinigungstextil kurzzeitig in die Desinfektionslösung eingetaucht, überschüssiges Desinfektionsmittel abgepresst und mit dem feuchten Bezug die desinfizierende Reinigung durchgeführt. In beiden Verfahren wird ein hohes Maß an hygienischer Sicherheit gewährleistet, da die Reinigungstextilien lediglich in einem Raum zur Anwendung kommen, danach als gebrauchtes Reinigungstextil abgelegt und anschließend desinfizierend gewaschen werden.
Beide beschriebenen Reinigungsverfahren sind jedoch sehr arbeitsintensiv, so dass nach vereinfachten Varianten gesucht wird. Ein derartiges günstigeres Verfahren stellt eine Modifikation des Ein-Bezugs-Verfahrens dar, in dem mehrere Reinigungstextilien in die Desinfektionslösung bei Arbeitsantritt eingelegt und bei Bedarf verwendet werden. Da dieses Verfahren mit einer wesentlichen Verringerung des Arbeitsaufwandes verbunden ist, wird sein Einsatz insbesondere in Krankenhäusern in hohem Maße forciert.

Bei der Prüfung der Desinfektionsmittelkonzentrationen bei diesem Verfahren wurde jedoch festgestellt, dass die Desinfektionsmittelkonzentration in der Reinigungslösung mit zunehmender Zeit erheblich abnimmt. Es wurde festgestellt, dass nach dem Einlegen der Reinigungstextilien in die Desinfektionslösung durch den längeren Kontakt der nicht sofort verwendeten Reinigungstextilien mit der Desinfektionslösung eine Adsorption von desinfizierenden Wirkstoffen an der relativ großen Oberfläche der Reinigungstextilien stattfindet. Diese Adsorption der desinfizierenden Wirkstoffe an den Reinigungstextilien ist von Textilart und Art der Wirkstoffe abhängig. Es wurde aber gefunden, dass im Mittel die meisten Reinigungstextilien eine derartige Adsorption zeigen. Es ist daher möglich, dass nach einer gewissen Zeit nur noch ein Bruchteil des desinfizierenden Wirkstoffs in der Anwendungslösung nachgewiesen werden kann, so dass die Reinigungslösung nach einer gewissen Zeit nicht mehr ausreichend desinfizierende Wirkung besitzt. Weiterhin kann selbst wenn an der Oberfläche des jeweils verwendeten Reinigungstextils noch ausreichend adsorbierter desinfizierter Wirkstoff vorhanden ist, dieser nicht mehr oder nur noch im geringen Maße auf die zu desinfizierenden Oberflächen aufgetragen werden, da er am Reinigungstextil adsorbiert ist und so nicht abgegeben wird. Diese Effekte verursachen eine unvollständige Desinfektion der zu behandelnden Flächen, so dass die notwendigen Hygieneanforderungen nicht mehr erfüllt werden.

Die Aufgabe der hier vorliegenden Erfindung ist es, die geschilderten Nachteile des Standes der Technik zu vermeiden. Es soll ein wässriges Konzentrat bereitgestellt werden, bei der in verdünnten Lösungen des wässrigen Konzentrats die enthaltenen antimikrobiellen Wirkstoffe nicht mehr in dem Maße an Reinigungstextilien adsorbiert werden, wie dies bei Desinfektionsmittellösungen des Standes der Technik erfolgt. Dies soll es vor allem ermöglichen Reinigungstextilien vor Gebrauch in desinfizierenden Lösungen zu lagern und dabei eine bessere Nutzung der eingesetzten antimikrobiellen Wirkstoffe für den ihnen originär obliegenden Verwendungszweck, die Desinfektion, zu gewährleisten.

Diese Aufgabe wird gelöst durch ein Verfahren zur Behandlung von Reinigungstextilien gemäß Anspruch 1.

Ein wesentlicher Vorteil des erfindungsgemäßen Verfahrens liegt darin, der Abnahme der Konzentration des genannten antimikrobiellen Wirkstoffes in Desinfektionsmittellösungen entgegenzuwirken, die dafür vorgesehen sind, von Reinigungstextilien aufgenommen und anschließend für die Flächendesinfektion verwendet zu werden. Dieser Effekt macht sich besonders stark bemerkbar, wenn der Kontakt (z.B. bei Lagerung) zwischen Desinfektionsmittellösungen und Reinigungstextilien lange andauert.

Reinigungstextilien im Sinne dieser Erfindung sind alle Hilfsmittel, die zur Desinfektion von Oberflächen zuerst mit der Desinfektionslösung und anschließend mit der zu desinfizierenden Oberfläche in Kontakt gebracht werden. Dies können Tücher, Lappen oder Vliese sein. Es ist aber auch möglich Schwämme oder Bürsten zu verwenden. Eine Ausführungsform ist ein Wischmop, der leicht wechselbare Bezüge aufweist. Die Reinigungstextilien können aus Fasern nativer Herkunft, wie z.B. Baumwolle hergestellt werden. Es ist aber auch möglich Fasern künstlicher Herkunft, wie z.B. Mikrofasern zu verwenden. Es ist grundsätzlich auch möglich mit Polymeren beschichtete Reinigungstextilien zu verwenden.

Der Effekt der hier vorliegenden Erfindung zeigt sich überraschenderweise bei den bereits genannten vielfach verwendeten antimikrobiellen Wirkstoffen. Aus der Wirkstoffgruppe der Aldehyde sind besonders Formaldehyd, Glyoxal, Glutaraldehyd und andere Derivate von Aldehyden hervorzuheben. Als weitere wichtige Wirkstoffgruppe wurden bereits die Phenole und Phenolderivate sowie die quartären Ammoniumverbindungen der Formel I genannt, von denen besonders diejenigen bevorzugt sind, in denen Dimethyl-didecyl-ammonium und/oder Dimethyl-dioctyl-ammonium und/oder Benzalkonium als kationische Komponente vorliegt.

Antimikrobielle Wirkstoffe im Sinne dieser Erfindung sind aber auch Amine bzw. Derivate von Aminen.

Als besonders vorteilhaft hat sich herausgestellt, wenn der genannte antimikrobielle Wirkstoff mit Amino-Gruppen ausgewählt ist aus

Alkylaminen der Formel (III) und/oder (IV)

R¹⁰-NH-(CH₂)₃NH₂ (III),

R¹⁰-N-[(CH₂)₃NH₂]₂ (IV),

die unneutralisiert, teilweise oder vollständig neutralisiert vorliegen können, wobei R¹⁰ für einen Alkylrest mit 8 bis 18 C-Atomen, vorzugsweise 12 bis 14 C-Atomen, steht, und/oder
Wirkstoffen, die durch Umsetzung eines Propylendiamins gemäß Formel (III),

R¹⁰-NH-(CH₂)₃NH₂ (III),

mit Glutaminsäure oder Glutaminsäurederivaten gemäß Formel (V),

R¹¹-O-CO-(CH₂)₂-CH(NH₂)-COOH (V),

in der R¹¹ Wasserstoff oder einen Alkylrest mit 1 bis 4 Kohlenstoffatomen bedeutet, und gegebenenfalls Umsetzung des so erhaltenen Produkts mit Ethylenoxid und/oder Propylenoxid und gegebenenfalls weitere Umsetzung mit organischen oder anorganischen Säuren erhältlich sind.

Amide gehören ebenfalls zur Klasse der antimikrobiellen Wirkstoffe gemäß der vorliegenden Erfindung. Die Pyrrolidoncarbonsäureamide sowie deren Salze, die beispielsweise durch die bereits angeführte Umsetzung von Aminen zugänglich sein können, treten hier in ihrer Wichtigkeit hervor. Besonders hervorzuheben sind hier die Umsetzungsprodukte von Glutaminsäure mit Alkylpropylendiaminen, die auch unter dem Handelsnamen Glucoprotamin^{®} bekannt sind.

Es ist selbstverständlich, dass sich die Vorteile des erfindungsgemäßen Verfahrens auch dann einstellen, wenn in dem Verfahren mehrere der genannten und/oder zusätzliche antimikrobielle Wirkstoffe nebeneinander vorliegen.

Die in dem erfindungsgemäßen Verfahren aufgeführten Komponenten der Formel II sind besonders bevorzugt ausgewählt aus der Gruppe der Difettsäuretrialkanolaminestersalze, die auch als Esterquats bezeichnet werden. Diese quartären Ammoniumverbindungen lassen sich durch die allgemeine Formel VI 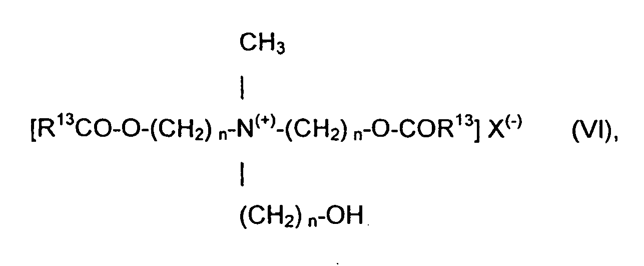 darstellen, in der R¹³CO für einen aliphatischen Acylrest mit 12-22 C-Atomen und 0, 1, 2 oder 3 Doppelbindungen, n für 2 oder 3 und X für Halogenid, Methoxysulfat oder Methoxyphosphat steht.

Wie bereits gesagt, ist es auch bevorzugt, dass das im erfindungsgemäßen Verfahren enthaltene Additiv auch kationische Polymere umfasst. Diese können zum einen Poly(dialkyldiallylammoniumsalze) oder deren Derivate sein, bzw. Copolymere von Dialkyldiallylammoniumsalzen mit Acrylamid und/oder Acrylsäure und/oder Vinylacetat und deren Derivaten. In einer besonderen Ausführungsform wird das Poly(dimethyldialkylammonium)chlorid oder ein Copolymer von Dimethyldiallylammoniumchlorid mit Acrylamid und/oder Acrylsäure und/oder Vinylacetat oder Derivate von Copolymeren von Dimethyldiallylammoniumchlorid mit Acrylamid und/oder Acrylsäure und/oder Vinylacetat verwendet. Beispiele für diese Verbindungen sind die von Chemviron vertriebenen kationischen Polymere, die unter dem Handelsnamen MERQUAT vertrieben werden.

Alle hier aufgezählten Additive können entweder als Einzelsubstanz oder aber in Gemischen untereinander eingesetzt werden, um die erfindungsgemäße Aufgabe zu lösen.

Als zusätzliche Formulierungsbestandteile sind insbesondere Ölsulfonate, Fettalkoholsulfate, Fettsäurekondensationsprodukte, Alkylpolyglykolether und Alkylarylpolyglykolether oder Gemische derselben bevorzugt.

In bevorzugter Weise kann das wässrige Konzentrat weitere Hilfs- und Zusatzstoffe aus den Gruppen der Tenside, Verlaufshilfsmittel, Komplexbildnersäuren, Säuren, organische Lösungsmittel, Lösungsvermittler, Farbstoffe, Duftstoffe und deren Gemische enthalten.

Aus den Konzentraten sind die in der praktischen Anwendung üblicherweise verwendeten Desinfektionslösungen beispielsweise durch einfaches Verdünnen mit Wasser zugänglich. Es sei allerdings ausdrücklich erwähnt, dass es auch möglich ist, zu den gewünschten wässrigen Desinfektionslösungen zu gelangen, ohne das Konzentrat einzusetzen.
Dementsprechend ist es das Anliegen der vorliegenden Anmeldung auch die Ausführungsformen zu umfassen, bei denen die in der praktischen Anwendung herangezogenen Desinfektionslösungen auf anderem Wege hergestellt werden. Beispielsweise ist es möglich, die im Konzentrat enthaltenen Komponenten a) (antimikrobieller Wirkstoff) und b) (Additiv) einzeln zu verdünnen und danach zusammenzubringen oder eine der Komponenten zu verdünnen und die andere in konzentrierter Form hinzuzugeben. Durch diese und gegebenenfalls andere übliche Vorgehensweisen gelangt der Fachmann ebenfalls zu den gewünschten wässrigen Desinfektionslösungen.

Im Verfahren wird eine wässrige Zubereitung verwendet, die in ihrer Zusammensetzung einer Desinfektionslösung entspricht, die durch Verdünnen des Konzentrats mit Wasser im Verhältnis 1:10 bis 1:400, vorzugsweise im Verhältnis 1:20 bis 1:200 und besonders bevorzugt im Verhältnis 1:30 bis 1:100 erhältlich ist.

In bevorzugter Weise können wie beschrieben der genannte antimikrobielle Wirkstoff und/oder das genannte Additiv separat einer vorliegenden wässrigen Lösung zugesetzt werden, wobei deren Menge so gewählt wird dass die schließlich vorliegende wässrige Zubereitung so viel des genannten antimikrobiellen Wirkstoffs und des genannten Additivs vorliegt, wie sich aufgrund der genannten Verdünnungsverhältnisse des Konzentrats ergibt. Es kann aber ebenso die Aufgabe der vorliegenden Erfindung gelöst werden, indem man Additiv und die wässrige Zubereitung des antimikrobiellen Wirkstoffes räumlich voneinander getrennt anwendet.

Das erfindungsgemäße Verfahren dient zur Verringerung des Wirkstoffverlustes in der wässrigen Zubereitung. Erläuternd sei an dieser Stelle darauf hingewiesen, dass in der vorliegenden Anmeldung unter einer wässrigen Zubereitung dasselbe zu verstehen ist, wie unter Desinfektionslösung.

Mit anderen Worten wird bei dem erfindungsgemäßen Verfahren das Reinigungstextil erst mit einem oder mehreren Additiven in verdünnter oder unverdünnter Form behandelt, wobei das oder die Additive ausgewählt sind aus der Gruppe der quartären Ammoniumverbindungen der Formel II 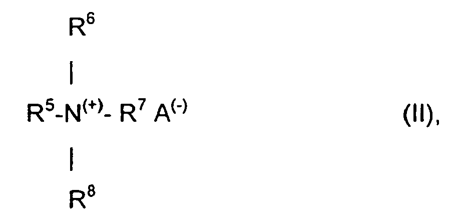 in der R⁵ und R⁶ Alkylgruppen mit 16-22 C-Atomen oder Gruppen der Formel R⁹CO (XCₙH₂ₙ)ₐ sind, worin R⁹CO eine lineare Acylgruppe mit 16-22 C-Atomen, X=Sauerstoff oder -NH-, n=2 oder 3, a=1 bis 4, R⁷ eine Gruppe gemäß R⁵ und R⁶ oder eine Alkylgruppe mit 1-4 C-Atomen und R⁸ eine Alkylgruppe mit 1-4 C-Atomen oder eine Hydroxyalkylgruppe mit 2-4 C-Atomen und A⁽⁻⁾ ein Halogenid-, Methoxysulfat- oder Methoxyphosphat-Anion ist,
aus den Poly-dialkyldiallylammoniumsalze und deren Derivaten, den Copolymeren von Dialkyldiallylammoniumsalzen mit Acrylamid und/oder Acrylsäure und/oder Vinylacetat und deren Derivaten und
danach mit der wässrigen Zubereitung behandelt.

Beispielsweise ist es möglich, dass in einer Ausführungsform das Reinigungstextil erst mit einer Lösung des genannten Additivs vorbehandelt wird. Dies kann z. B. dadurch geschehen, dass das Reinigungstextil in eine Vorbehandlungs-Lösung des Additivs eingetaucht wird. Gewünschtenfalls kann das Reinigungstextil nach dem Eintauchen getrocknet werden. Wenn ein so vorbehandeltes Reinigungstextil danach in eine wässrige Zubereitung eingetaucht wird, so wird der in der wässrigen Zubereitung enthaltene antimikrobielle Wirkstoff nicht mehr in dem Ausmaße von dem Reinigungstextil adsorbiert, wie dies ohne entsprechende Vorbehandlung der Fall ist.
Daraus ergibt sich für die praktische Anwendung als erheblicher Vorteil, dass bei längerer Lagerung von Reinigungstextilien in Desinfektionslösungen keine, oder aber nur minimale Abnahme der Wirksamkeit der Desinfektionslösung beobachtet wird.

Bei dem erfindungsgemäßen Verfahren ist es bevorzugt, wenn das oder die genannten Additive bei der Vorbehandlung insgesamt eine Konzentration von 0,01 bis 10 Gew.-%, besonders bevorzugt 0,05 bis 1 Gew.-% und ganz besonders bevorzugt 0,075 bis 0,15 Gew.-%, bezogen auf die Vorbehandlungslösung, ausmachen.

Die vorliegende Erfindung soll an den folgenden Beispielen erläutert werden, ist aber nicht auf diese beschränkt.

### Beispiele

Zur Durchführung der Beispiele wurden verschiedene wässrige Desinfektionslösungen herangezogen.
Die Lösung E1 enthielt als antimikrobielle Wirkstoffe etwa 0,05 Gew.-% Glutaraldehyd sowie etwa 0,1 Gew.-% Glyoxal, jeweils bezogen auf die gesamte Lösung, neben weiteren für die vorliegende Erfindung nicht wesentlichen Formulierungsbestandteilen.
Die Lösung E2 enthielt als antimikrobielle Wirkstoffe etwa 0,15 Gew.-% Benzalkoniumchlorid sowie etwa 0,02 Gew.-% 2-Phenylphenol und etwa 0,02 Gew.-% Polyhexamethylenbiguanidiniumchlorid, jeweils bezogen auf die gesamte Lösung, neben weiteren für die vorliegende Erfindung nicht wesentlichen Formulierungsbestandteilen.
Die Lösung E3 enthielt als antimikrobielle Wirkstoffe etwa 0,13 Gew.-% Glucoprotamin^{®} (Handelsprodukt der Ecolab GmbH & CO OHG) sowie etwa 0,01 Gew.-% Phenoxyethanol, jeweils bezogen auf die gesamte Lösung, neben weiteren für die vorliegende Erfindung nicht wesentlichen Formulierungsbestandteilen.

Als Reinigungstextil wurde einerseits ein Polyfix^{®}-Tuch, das aus einer mit Polyurethan getränkten Viskose/Vlies-Matrix besteht, und ein Polyfix^{®} microclean-Tuch, das aus einer Mikrofaser besteht, eingesetzt. Beide Reinigungstextilien sind Handelswaren der Ecolab GmbH & CO OHG. Als Additiv wurde Poly(dimethyldiallylammoniumchlorid) verwendet, das unter dem Handelsnamen Polyquat^{®} 40 erhältlich ist.

Die Reinigungstextilien wurden in einer herkömmlichen Waschmaschine bei 60°C mit einem üblichen Textilreinigungsmittel in üblicher Weise gewaschen.

Abweichend zu dem üblichen Waschvorgang wurde bei der erfindungsgemäßen Testdurchführung in den letzten Spülgang der Waschmaschine, der sonst nur mit Wasser durchgeführt wird, 3,0 g/l Polyquat^{®} 40 (Additiv) zugegeben. Nach dem Waschen wurden die Tücher 3 h bei Raumtemperatur getrocknet. Anschließend wurden die 8-fach gefalteten Tücher für 60 min in die jeweiligen Desinfektionslösungen eingelegt. Nach Ablauf dieser Zeit wurde das Reinigungstextil von immer derselben Person unter etwa denselben Bedingungen ausgewrungen und die resultierende Lösung aufgefangen. Hieraus wurde der Wirkstoffverlust an Desinfektionsmittel in der so resultierenden Lösung bestimmt.

Die Ergebnisse der Untersuchungen können aus Tabelle 1 entnommen werden. Zur Erläuterung sei gesagt, dass bei den Versuchen ermittelt wurde, um wie viel die nach dem 60-minütigen Einlegen der Tücher frei verfügbare Wirkstoffkonzentration von der Wirkstoffkonzentration abweicht, die zu Beginn der Versuche in der jeweiligen Desinfektionslösung vorlag. Die Angabe in der Tabelle erfolgten als prozentuale Abweichung, wobei die Anfangs-Wirkstoffkonzentration gleich 100% gesetzt wurde.

**Tabelle 1:**

| Untersuchungen zum Wirkstoffverlust in Desinfektionslösungen bei unterschiedlichen Bedingungen | | | | |
|---|---|---|---|---|
| | Wirkstoffverlust bei Polyfix^{®} microclean-Tuch | | Wirkstoffverlust bei Polyfix^{®} Tuch | |
| Desinfektionslösung | Ohne Behandlung im letzten Spülgang | Mit Zugabe Polyquat^{®} 40 im letzten Spülgang | Ohne Behandlung im letzten Spülgang | Mit Zugabe Polyquat^{®} 40 im letzten Spülgang |
| E1 | 31 % | 5% | 31 % | 9% |
| E2 | 56 % | 23 % | 31 % | 12 % |
| E3 | 30% | 9% | 35 % | 8% |

Anhand der Tabelle 1 kann man den Effekt, den der Einsatz des Additivs bewirkt, erkennen. Unabhängig von der Beschaffenheit des Reinigungstextils und des Desinfektionsmittels wurde der Wirkstoffverlust an Desinfektionsmittel in der aufgefangenen Desinfektionslösung durch die Zugabe des Additivs deutlich verringert.

Ähnliche Ergebnisse werden auch erreicht, wenn antimikrobieller Wirkstoff und Additiv in einer Lösung vorliegen.

## Patentansprüche

1. Verfahren zur Verringerung des Wirkstoffverlustes von Desinfektionsmittellösungen, die in Kontakt mit Reinigungstextilien kommen, wobei das Verfahren eine Behandlung der Renigungstextilien umfasst, bei der das Reinigungstextil
a) erst in einer Vorbehandlung mit einem oder mehreren Additiv(en) aus der Gruppe
i) der quartären Ammoniumverbindungen der Formel II 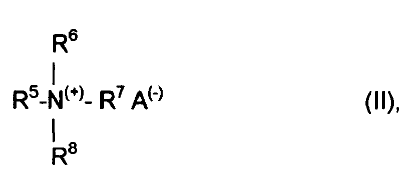 in der R⁵ und R⁶ Alkylgruppen mit 16-22 C-Atomen oder Gruppen der Formel R⁹CO (XCₙH₂ₙ)ₐ sind, worin R⁹CO eine lineare Acylgruppe mit 16-22 C-Atomen, X=Sauerstoff oder -NH-, n=2 oder 3, a=1 bis 4, R⁷ eine Gruppe gemäß R⁵ und R⁶ oder eine Alkylgruppe mit 1-4 C-Atomen und R⁸ eine Alkylgruppe mit 1-4 C-Atomen oder eine Hydroxyalkylgruppe mit 2-4 C-Atomen und A⁽⁻⁾ ein Halogenid-, Methoxysulfat- oder Methoxyphosphat-Anion ist,
ii) aus den Polydialkyldiallylammoniumsalze und deren Derivaten,
iii) den Copolymeren von Dialkyldiallylammoniumsalzen mit Acrylamid und/oder Acrylsäure und/oder Vinylacetat und deren Derivaten
in verdünnter oder unverdünnter Form behandelt wird, und danach
b) mit einer Desinfektionslösung behandelt wird, wobei die Desinfektionslösung aus einem Konzentrat enthaltend
i) 5 bis 30 Gew.-% eines antimikrobiellen Wirkstoffes ausgewählt aus der Gruppe der Aldehyde und deren Derivate, Phenole, Phenolderivate, Amide, Derivate von Amiden, Amine, Derivate von Aminen, der quartären Ammoniumverbindungen der Formel I 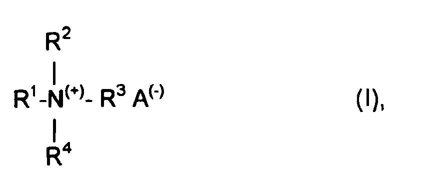 in der R¹ eine Alkylgruppe mit 6-16 C-Atomen, R² eine Alkylgruppe mit 1-12 C-Atomen oder eine Benzylgruppe, R³ und R⁴ Alkylgruppen mit 1-4 C-Atomen oder Hydroxyalkylgruppen mit 2-4 C-Atomen und A⁽⁻⁾ ein Halogenid-, Methoxysulfat- oder Methoxyphosphat-Anion ist, und
ii) 5 bis 50 Gew.-% eines Additivs ausgewählt aus der unter a) i) bis iii) genannten Gruppe
durch Verdünnen mit Wasser im Verhältniss 1:10 bis 1:400 erhältlich ist.

2. Verfahren nach Anspruch 1, **dadurch gekennzeichnet, dass** die genannten antimikrobiellen Wirkstoffe ausgewählt sind aus Formaldehyd, Glutaraldehyd, Glyoxal, aus den quartären Ammoniumverbindungen mit Dimethyl-didecyl-ammonium und/oder Dimethyl-dioctyl-ammonium und/oder Benzalkonium als kationischer Komponente, aus den Aminen bzw. Derivaten von Aminen umfassend Alkylamine der Formel (III) und/oder (IV)
R¹⁰-NH-(CH₂)₃NH₂ (III),
R¹⁰-N-[(CH₂)₃NH₂]₂ (IV),
die unneutralisiert, teilweise oder vollständig neutralisiert vorliegen können, wobei R¹⁰ für einen Alkylrest mit 8 bis 18 C-Atomen, vorzugsweise 12 bis 14 C-Atomen, steht, und/oder
Wirkstoffen, die durch Umsetzung eines Propylendiamins gemäß Formel (III),
R¹⁰-NH-(CH₂)₃NH₂ (III),
mit Glutaminsäure oder Glutaminsäurederivaten gemäß Formel (V),
R¹¹-O-CO-(CH₂)₂-CH(NH₂)-COOH (V),
in der R¹¹ Wasserstoff oder einen Alkylrest mit 1 bis 4 Kohlenstoffatomen bedeutet, und gegebenenfalls Umsetzung des so erhaltenen Produkts mit Ethylenoxid und/oder Propylenoxid und gegebenenfalls weitere Umsetzung mit organischen oder anorganischen Säuren erhältlich sind, aus den Amiden, vorzugsweise Pyrrolidoncarbonsäureamiden sowie deren Salzen, insbesondere den Umsetzungsprodukten von Glutaminsäure mit Alkylpropylendiaminen.

3. Verfahren nach Anspruch 1 oder 2, **dadurch gekennzeichnet, dass** die als Additiv genannten Verbindungen der Formel II ausgewählt sind aus der Gruppe der Difettsäuretrialkanolaminestersalze der allgemeinen Formel VI 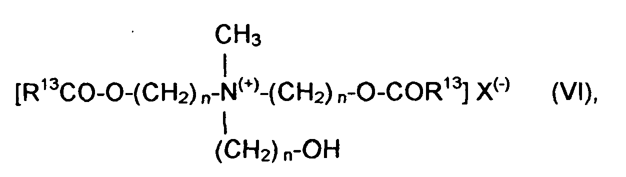 in der R¹³CO für einen aliphatischen Acylrest mit 12-22 C-Atomen und 0, 1, 2 oder 3 Doppelbindungen, n für 2 oder 3 und X für Halogenid, Methoxysulfat oder Methoxyphosphat steht, sowie den kationischen Polymeren umfassend Poly(dialkyldiallylammoniumsalze) oder deren Derivate, bzw. Copolymere von Dialkyldiallylammoniumsalzen mit Acrylamid und/oder Acrylsäure und/oder Vinylacetat und deren Derivaten oder Mischungen derselben.

4. Verfahren nach einem oder mehreren der Anspruche 1 bis 3, **dadurch gekennzeichnet, dass** insgesamt 10 bis 25 Gew.-% des genannten antimikrobiellen Wirkstoffes, bezogen auf das gesamte Konzentrat, enthalten sind.

5. Verfahren nach einem oder mehreren der Anspruche 1 bis 4, **dadurch gekennzeichnet, dass** 10 bis 40 % Gew.-% des genannten Additivs, bezogen auf das gesamte Konzentrat, enthalten sind.

6. Verfahren nach einem oder mehreren der Anspruche 1 bis 5, **dadurch gekennzeichnet, dass** das Konzentrat weitere Hilfs- und Zusatzstoffe aus der Gruppe Tenside, Verlaufshilfsmittel, Komplexbildnersäuren, Säuren, organische Lösungsmittel, Lösungsvermittler, Farbstoffe, Duftstoffe und deren Gemische enthält.

7. Verfahren nach einem oder mehreren der Anspruche 1 bis 6, **dadurch gekennzeichnet, dass** die Verdünnung mit Wasser 1:20 bis 1:200 ist.

8. Verfahren nach einem oder mehreren der Anspruche 1 bis 7, **dadurch gekennzeichnet, dass** man bei der Herstellung der genannten Zubereitung, von dem genannten antimikrobiellen Wirkstoff und dem genannten Additiv in getrennter Form anstatt in Konzentratform ausgegangen wird.

9. Verfahren nach einem oder mehreren der Anspruche 1 bis 8, **dadurch gekennzeichnet, dass** die Behandlung mit einer wässrigen Lösung des Additivs erfolgt.

10. Verfahren nach Anspruch 9, **dadurch gekennzeichnet, dass**, **dadurch gekennzeichnet, dass** das Additiv in einer Konzentration von 0,01 bis 10 Gew.-% eingesetzt wird.

11. Verfahren nach Anspruch 10, **dadurch gekennzeichnet, dass** das Additiv in einer Konzentration von 0,05 bis 1 Gew.-% eingesetzt wird.

## Claims

1. A process for reducing the loss of active substance of disinfectant solutions coming into contact with cleaning textiles, wherein said process comprises a treatment of said cleaning textiles, in which the cleaning textiles
a) are first treated in a pretreatment stage with one or more additive(s) from the group of
i) the quaternary ammonium compounds of Formula (II) 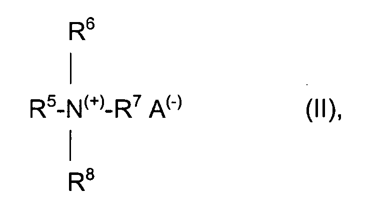 where R⁵ and R⁶ are alkyl groups having 16-22 carbon atoms or groups of the formula R⁹CO(XCₙH₂ₙ)ₐ, where R⁹CO is a linear acyl group having 16-22 carbon atoms, X is oxygen or -NH-, n is 2 or 3, a is 1 to 4, R⁷ is a group corresponding to R⁵ and R⁶ or an alkyl group having 1-4 carbon atoms, and R⁸ is an alkyl group having 1-4 carbon atoms or a hydroxyl alkyl group having 2-5 carbon atoms, and A⁽⁻⁾ is a halide anion, a methoxysulfate anion or a methoxyphosphate anion,
ii) the poly(dialkyl diallyl ammonium) salts and derivatives thereof,
iii) the copolymers of dialkyl diallyl ammonium salts with acrylamide and/or acrylic acid and/or vinyl acetate and derivatives thereof,
in diluted or undiluted form, and afterwards
b) are treated with a disinfectant solution, wherein said disinfectant solution is obtainable from a concentrate containing
i) 5 to 30% by weight of an antimicrobial agent selected from the group consisting of aldehydes and derivatives thereof, phenols, phenol derivatives, amides, amide derivatives, amines, amine derivatives, the quaternary ammonium compounds of Formula (I) 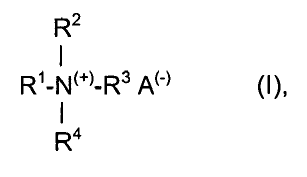 where R¹ is an alkyl group having 6-16 carbon atoms, R² is an alkyl group having 1-12 carbon atoms or a benzyl group, R³ and R⁴ are alkyl groups having 1-4 carbon atoms or hydroxy alkyl groups having 2-4 carbon atoms, and A⁽⁻⁾ is a halide anion, a methoxysulfate anion or a methoxyphosphate anion, and
ii) 5 to 50% by weight of an additive selected from the group described under a) i) to iii)
by diluting with water at a ratio of 1:10 to 1:400.

2. The process according to Claim 1, wherein said antimicrobial agents are selected from the group consisting of formaldehyde, glutaraldehyde, glyoxal, from the group of quaternary ammonium compounds consisting of dimethyl didecyl ammonium and/or dimethyl dioctyl ammonium and/or benzalkonium as cationic component, from the group of amines or amine derivatives, respectively, comprising alkyl amines corresponding to Formulas (III) and/or (IV)
R¹⁰-NH-(CH₂)₃NH₂ (III),
R¹⁰-N-[(CH₂)₃NH₂]₂ (IV),
that may be present in unneutralized, partially neutralized or completely neutralized form, where R¹⁰ represents an alkyl moiety having 8 to 10 carbon atoms, preferably 12 to 14 carbon atoms, and/or
active substances that are obtainable by reacting a propylene diamine corresponding to Formula (III),
R¹⁰-NH-(CH₂)₃NH₂ (III),
with glutamic acid or glutamic acid derivatives, corresponding to Formula (V),
R¹¹-O-CO-(CH₂)₂-CH(NH₂)-COOH (V),
where R¹¹ represents hydrogen or an alkyl moiety having 1 to 4 carbon atoms, and, if required, reacting the product obtained in this way with ethylene oxide and/or propylene oxide and, if required, further reacting with organic or inorganic acids, from the group of amides, preferably amides of pyrrolidon carboxylic acids and salts thereof, particularly the reaction products of glutamic acid with alkyl propylene diamines.

3. The process according to Claim 1 or 2, wherein said compounds of Formula (II) mentioned as additive are selected from the group of the difatty trialkanolamine ester salts of the General Formula (VI) 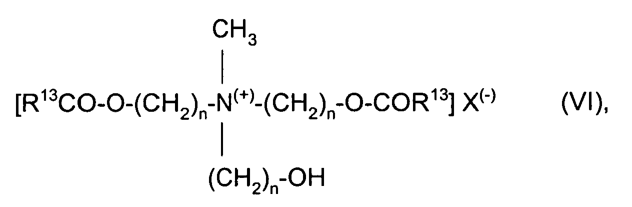 where R¹³CO represents an aliphatic acyl moiety having 12-22 carbon atoms and 0, 1, 2 or 3 double bonds, n represents 2 or 3 and X is halide, methoxysulfate or methoxyphosphate, and from the group of cationic polymers, comprising poly(dialkyl diallyl ammonium) salts or derivatives thereof, or copolymers of dialkyl diallyl ammonium salts with acrylamide and/or acrylic acid and/or vinyl acetate and derivatives thereof, or mixtures thereof, respectively.

4. The process according to one or more of Claims 1 to 3, **characterized in that** a total of 10 to 25% by weight of said antimicrobial agent is present, based on the concentrate as a whole.

5. The process according to one or more of Claims 1 to 4, **characterized in that** 10 to 40% by weight of said additive are present, based on the concentrate as a whole.

6. The process according to one or more of Claims 1 to 5, wherein said concentrate contains other adjuvants and additives from the group consisting of surfactants, flow agents, complex forming acids, acids, organic solvents, solubilizers, colorants, fragrances, and mixtures thereof.

7. The process according to one or more of Claims 1 to 6, **characterized in that** the dilution with water is 1:20 to 1:200.

8. The process according to one or more of Claims 1 to 7, wherein said preparation is prepared by using said antimicrobial agent and said additive separately instead of using the concentrate.

9. The process according to one or more of Claims 1 to 8, **characterized in that** the treatment is performed with an aqueous solution of the additive.

10. The process according to Claim 9, wherein said additive is employed in a concentration of 0.01 to 10% by weight.

11. The process according to Claim 10, wherein said additive is employed in a concentration of 0.05 to 1 % by weight.

## Revendications

1. Procédé destiné à réduire la perte de substances actives de solutions désinfectantes, qui entrent en contact avec des tissus de nettoyage, le procédé comprenant un traitement des tissus de nettoyage, lors duquel le tissu de nettoyage
a) est tout d'abord traité dans un prétraitement avec un ou plusieurs additif(s) choisis dans le groupe
i) des composés ammonium quaternaires de formule II 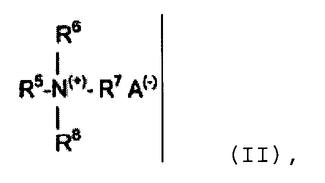 dans laquelle R⁵ et R⁶ représentent des groupes alkyles avec 16 à 22 atomes de carbone ou des groupes de formule R⁹CO (XCₙH₂ₙ)ₐ, dans laquelle R⁹CO représente un groupe acyle linéaire avec 16 à 22 atomes de carbone, X représente l'oxygène ou -NH-, n = 2 ou 3, a = 1 à 4, R⁷ représente un groupe selon R⁵ et R⁶ ou un groupe alkyle avec 1 à 4 atomes de carbone et R⁸ représente un groupe alkyle avec 1 à 4 atomes de carbone ou un groupe hydroxyalkyle avec 2 à 4 atomes de carbone et A⁽⁻⁾ représente un anion halogénure, méthoxysulfate ou méthoxyphosphate,
ii) des sels de polydialkyldiallylammonium et de leurs dérivés,
iii) des copolymères de sels de dialkyldiallylammonium avec l'acrylamide et/ou l'acide acrylique et/ou l'acétate de vinyle et de leurs dérivés sous forme diluée ou non diluée, et ensuite
b) est traité avec une solution désinfectante, la solution désinfectante pouvant être obtenue à partir d'un concentré contenant
i) de 5 à 30 % en poids d'une substance active antimicrobienne choisie dans le groupe des aldéhydes et de leurs dérivés, des phénols, des dérivés de phénols, des amides, des dérivés d'amides, des amines, des dérivés d'amines, des composés ammonium quaternaires de formule I 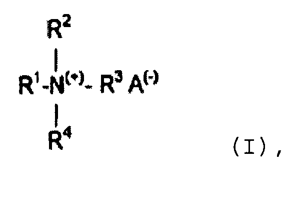 dans laquelle R¹ représente un groupe alkyle avec 6 à 16 atomes de carbone, R² représente un groupe alkyle avec 1 à 12 atomes de carbone ou un groupe benzyle, R³ et R⁴ représentent des groupes alkyles avec 1 à 4 atomes de carbone ou des groupes hydroxyalkyles avec 2 à 4 atomes de carbone et A⁽⁻⁾ représente un anion halogénure, méthoxysulfate ou méthoxyphosphate, et
ii) de 5 à 50 % en poids d'un additif choisi dans les groupes mentionnés sous a) i) à iii)
par dilution avec de l'eau dans une proportion de 1:10 à 1:400.

2. Procédé selon la revendication 1, **caractérisé en ce que**, les substances antimicrobiennes mentionnées sont choisies parmi le formaldéhyde, le glutaraldéhyde, le glyoxal, parmi les composés ammonium quaternaires avec le diméthyl-didécylammonium et/ou le diméthyldioctylammonium et/ou du benzalkonium comme composant cationique, parmi les amines respectivement les dérivés d'amines comprenant l'alkylamine de formule (III) et/ou (IV)
R¹⁰-NH-(CH₂)₃NH₂ (III),
R¹⁰-N-[(CH₂)₃NH₂] (IV),
qui peuvent se présenter sous forme non neutralisée, partiellement ou totalement neutralisée, R¹⁰ représentant un résidu alkyle avec 8 à 18 atomes de carbone, de préférence 12 à 14 atomes de carbone, et/ou
des substances actives, qui peuvent être obtenues par la conversion d'une propylènediamine selon la formule (III),
R¹⁰-NH- (CH₂)₃NH₂ (III),
avec un acide glutamique ou des dérivés d'acide glutamique selon la formule (V),
R¹¹-O-CO-(CH₂)₂-CH (NH₂) -COOH (V),
dans laquelle R¹¹ représente l'hydrogène ou un résidu alkyle avec 1 à 4 atomes de carbone, et le cas échéant la conversion du produit ainsi obtenu avec de l'oxyde d'éthylène et ou de l'oxyde de propylène et le cas échéant une conversion supplémentaire avec des acides organiques ou inorganiques, à partir des amides, de préférence les amides d'acides pyrrolidone-carboxyliques ainsi que leurs sels, en particulier les produits de conversion d'acides glutamiques avec des alkylpropylènediamines.

3. Procédé selon la revendication 1 ou 2, **caractérisé en ce que**, les composés de formule II mentionnés comme additifs sont choisis dans le groupe des sels de trialcanolamine-esters de diacides gras de formule générale VI 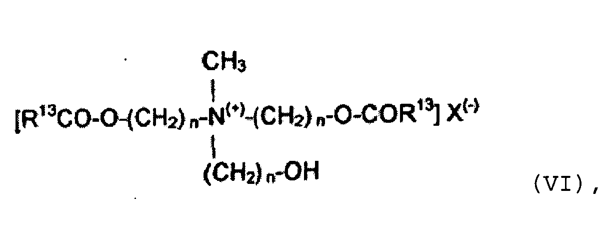 dans laquelle R¹³CO représente un résidu acyle aliphatique avec 12 à 22 atomes de carbone et 0, 1, 2 ou 3 doubles liaisons, n vaut 2 ou 3 et X représente un halogénure, un méthoxysulfate ou un méthoxyphosphate, ainsi que des polymères cationiques comprenant les sels de poly(dialkyldiallylammonium) ou leurs dérivés, respectivement des copolymères de sels de dialkyldiallylammonium avec l'acrylamide et/ou l'acide acrylique et/ou l'acétate de vinyle et de leurs dérivés ou de mélanges de ceux-ci.

4. Procédé selon l'une ou plusieurs des revendications 1 à 3, **caractérisé en ce que**, sont contenus au total de 10 à 25 % en poids de la substance active antimicrobienne mentionnée, basé sur le poids total du concentré.

5. Procédé selon l'une ou plusieurs des revendications 1 à 4, **caractérisé en ce que**, sont contenus de 10 à 40 % en poids de l'additif mentionné, basé sur le poids total du concentré.

6. Procédé selon l'une ou plusieurs des revendications 1 à 5, **caractérisé en ce que**, le concentré contient en outre d'autres substances auxiliaires ou additionnelles choisies dans le groupe des tensioactifs, des agents d'étalement, des acides complexants, des acides, des solvants organiques, des solubilisants, des colorants, des parfums et de leurs mélanges.

7. Procédé selon l'une ou plusieurs des revendications 1 à 6, **caractérisé en ce que**, la dilution avec de l'eau est de 1:20 à 1:200.

8. Procédé selon l'une ou plusieurs des revendications 1 à 7, **caractérisé en ce que**, lors de la fabrication de la préparation mentionnée, on part de la substance active antimicrobienne mentionnée et de l'additif mentionné sous forme séparée plutôt que sous forme de concentré.

9. Procédé selon l'une ou plusieurs des revendications 1 à 8, **caractérisé en ce que**, le traitement est effectué avec une solution aqueuse de l'additif.

10. Procédé selon la revendication 9, **caractérisé en ce que** l'additif est mis en oeuvre à une concentration de 0,01 à 10 % en poids.

11. Procédé selon la revendication 10, **caractérisé en ce que** l'additif est mis en oeuvre à une concentration de 0,05 à 1 % en poids.
